# EUROPÄISCHE PATENTSCHRIFT

(11) **EP 1 721 587 B1**
(45) Veröffentlichungstag und Bekanntmachung des Hinweises auf die Patenterteilung: **23.02.2011**
(21) Anmeldenummer: 06113230.4
(22) Anmeldetag: 27.04.2006
(51) Int. Cl.: A61F 5/00

(54) **Medizinische Vorrichtung zur Veränderung der Form menschlicher Hohlorgane**
Medical device for the modification of the shape of human hollow organs
Dispositif médical pour la modification de la forme d'organes creux humains

(30) Priorität: 13.05.2005 DE 202005007597 U; 30.08.2005 DE 102005041093
(43) Veröffentlichungstag der Anmeldung: 15.11.2006
(73) Patentinhaber: Ovesco Endoscopy GmbH, 72074 Tübingen (DE)
(72) Erfinder: Schurr, Marc Oliver Prof.Dr.med., 72072 Tübingen (DE); Ho, Chi-Nghia Dipl.-Ing., 70180 Stuttgart (DE)
(74) Vertreter: Winter, Brandl, Fürniss, Hübner, Röss, Kaiser, Polte - Partnerschaft

(56) Entgegenhaltungen:
- WO-A-01/35832
- WO-A-2004/087014
- DE-A1- 10 159 470
- US-A1- 2004 107 004
- US-A1- 2004 147 942

## Beschreibung

Die vorliegende Erfindung betrifft eine medizinische Vorrichtung zur Veränderung der Form menschlicher Hohlorgane, beispielsweise des Verdauungstrakts insbesondere des Magens, gemäß dem Oberbegriff des Patentanspruchs 1, wie die beispielsweise aus WO 01/35832 A2 bekannt ist.

Fettleibigkeit ist ein aktuelles Zivilisationsproblem, welches in den seltensten Fällen auf hormonelle Erkrankungen zurückzuführen ist. In den meisten Fällen sind falsche Essgewohnheiten, Bewegungsmangel, kalorienreiche Lebensmittel etc. die Hauptursachen von Fettsucht. Diäten sind zwar eine erfolgversprechende Möglichkeit der temporären und schnellen Reduzierung von Körperfett, sie bedingen jedoch einer gewissen Disziplin des betreffenden Patienten sowie einer permanenten Überwachung durch geschultes Fachpersonal.

An dieser Stelle sei darauf hingewiesen, dass erfahrungsgemäß gerade solche Essgewohnheiten, die zu Fettleibigkeit führen, auf undiszipliniertem Essverhalten seitens der betreffenden Patienten beruhen. Aus diesem Grund werden Diäten von diesen Patienten häufig nicht durchgehalten oder die Patienten fallen nach Beendigung einer Diät in ihre alten Essgewohnheiten zurück. In jedem Fall führen konventionelle Diäten in der Praxis zumeist nicht zu den gewünschten Ergebnissen.

Aus dem Stand der Technik ist es daher zur Lösung der vorstehenden Problematik bekannt geworden, Bereiche des Verdauungstrakts, insbesondere den Magen durch einen chirurgischen Eingriff zu verkleinern. Der Patient erfährt hierdurch bereits mit Aufnahme relativ kleiner Nahrungsportionen ein Völlegefühl, was ihn letztlich von weiterer Nahrungsaufnahme abhält, der Patient nimmt daher ab. Hierbei haben sich prinzipiell die nachfolgend kurz erläuterten Methoden zur Veränderung der Form und Größe des betreffenden menschlichen Organs durchgesetzt:
1. Die eine Methode sieht vor, das betreffende Organ, beispielsweise den Magen zu verkleinern, indem ein Teil des Magens vom übrigen Teil durch Tackern oder Nähen isoliert wird und somit zur Nahrungsaufnahme nicht mehr zur Verfügung steht. Obgleich sich durch diese Methode ein langanhaltender Erfolg bei der Bekämpfung von Fettsucht einstellt, ist sie insofern nachteilig, als dass der genannte Eingriff irreversibel und für den Patienten auch belastend ist.
2. Eine andere Methode sieht die Schaffung einer Abteilung des Magens durch ein von außen angelegtes Magenband vor. Dadurch wird ein Vormagen geschaffen, der sich rasch mit Nahrung füllt, womit ein schnelleres Sättigungsgefühl eintritt.
3. Auch bei anderen Krankheiten des Verdauungstraktes, z.B. bei der Refluxkrankheit der Speiseröhre spielt die Veränderung des Lumens des Hohlorgans für die Behandlung eine wichtige Rolle. So wird z.B. bei der Fundoplikatio der Mageneingang von außen durch eine Gewebemanschette oder ein künstliches Implantat gestützt.

Angesichts dieses Stands der Technik ist es eine Aufgabe der vorliegenden Erfindung, eine Vorrichtung zur Veränderung der Form menschlicher Hohlorgane zu schaffen, welche in einer den Patienten möglichst gering belastenden Weise einsetzbar ist und darüber hinaus die natürlichen Funktionen des betreffenden Organs weitgehend erhält.

Diese Aufgabe wird durch eine gattungsgemäße Vorrichtung mit den Merkmalen des Patentanspruchs 1 gelöst. Weitere vorteilhafte Ausgestaltungen der Erfindung sind Gegenstand der übrigen Unteransprüche.

Die Erfindung wird nachstehend anhand bevorzugter Ausführungsbeispiele unter Bezugnahme auf die begleitenden Zeichnungen näher erläutert.
Fig. 1 zeigt eine prinzipielle Darstellung einer medizinischen Vorrichtung gemäß einem ersten bevorzugten Ausführungsbeispiel der Erfindung,
Fig. 2 zeigt in vergrößerter Darstellung die erste Variante einer Gewebeklammer oder Clip, wie sie bei der Vorrichtung gemäß Fig. 1 Verwendung findet,
Fig. 3 zeigt in vergrößerter Darstellung die zweite Variante einer Gewebeklammer oder Clip, wie sie bei der Vorrichtung gemäß Fig. 1 Verwendung findet,
Fig. 4 zeigt in vergrößerter Darstellung die dritte Variante einer Gewebeklammer oder Clip, wie sie bei der Vorrichtung gemäß Fig. 1 Verwendung findet,
Fig. 5 zeigt in vergrößerter Darstellung nicht die Erfindung, sondern die erste Variante eines Zugankers als Alternative zu einer Gewebeklammer oder Clip, wie sie bei der Vorrichtung gemäß Fig. 1 Verwendung findet
Fig. 6 zeigt in vergrößerter Darstellung nicht die Erfindung, sondern die zweite Variante eines Zugankers als Alternative zu einer Gewebeklammer oder Clip, wie sie bei der Vorrichtung gemäß Fig. 1 Verwendung findet,
Fig. 7 und 8 zeigen jeweils eine prinzipielle Darstellung der medizinischen Vorrichtung gemäß dem ersten bevorzugten Ausführungsbeispiel der Erfindung unter Verwendung unterschiedlicher Clips gemäß der Fig. 3 und 4 und
Fig. 9 und 10 zeigen jeweils eine medizinische Vorrichtung gemäß einem zweiten bevorzugten Ausführungsbeispiel der Erfindung jedoch mit unterschiedlichem Bandverläufen.

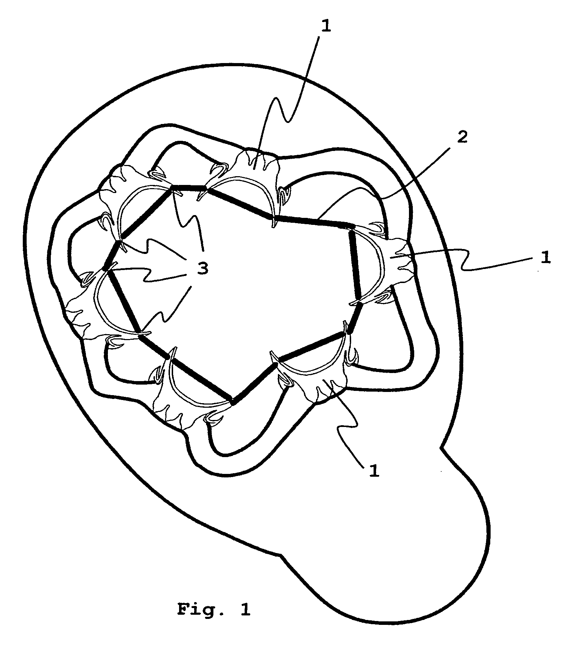

In der Fig. 1 ist eine medizinische Vorrichtung gemäß dem ersten bevorzugten Ausführungsbeispiel der Erfindung in Funktion dargestellt.

Demzufolge besteht die medizinische Vorrichtung im Wesentlichen aus einer Anzahl von Gewebeklammern 1, Clips oder Anker sowie einem vorzugsweise flexiblen ggf. auch elastischen Band 2, Draht oder Schlauch, an welchem die Clips 1 oder Anker aufgereiht sind.

Hierfür weist jeder Clip oder Anker eine Art Öse oder Durchgang 3 auf, durch welchen das Band 2 hindurchgezogen ist. Alternativ kann jeder Clip 1 oder Anker mit einer lösbaren Einhängvorrichtung beispielsweise in Form eines Schäckels, Hakens und dergl. (nicht weiter dargestellt) versehen sein, an welcher der Clip 1 an dem Band 2 befestigbar ist. Weiter vorzugsweise kann diese Einhängvorrichtung so ausgebildet sein, dass der Clip 1 oder Anker längs des Bands 2 verschiebbar ist.

Das in der Fig.1 dargestellte Band 2 besteht in einer bevorzugten Ausgestaltung aus einem vorzugsweise körperverträglichen flexiblen Material wie Nylon, das an seinen freien Enden entweder verknotet oder verschweißt werden kann oder aber über einen an den freien Enden des Bands angeordneten Verschlussmechanismus zu einem geschlossenen Ring bzw. zu einer Schlaufe verbunden werden kann. Der Verschlussmechanismus (nicht dargestellt) ist dabei vorzugsweise so ausgeführt, dass der geschlossene Ring bzw. die Schlaufe im Durchmesser etwas nach Art eines Gürtelschlosses verstellt und in einer frei gewählten Größe arretiert werden kann.

Anstelle des flexiblen Bands 2 ist es auch möglich, einen biegbaren Draht oder einen Federdraht vorzugsweise unter Verwendung des vorstehend beschriebenen Verschlussmechanismus vorzusehen. Auch ist die alternative Verwendung eines aufblasbaren flexiblen sowie ggf. elastischen Schlauchs denkbar, der zu einem geschlossenen oder offenen Ring bzw. einer Schlaufe geformt ist und der mit einem Gas, oder einem vorzugsweise körperverträglichen Fluid wie einer Kochsalzlösung oder einfach nur Wasser befüllbar ist, um hierdurch den vom Schlauch aufgespannten Ring- bzw. Schlaufendurchmesser zu vergrößern/verkleinern.

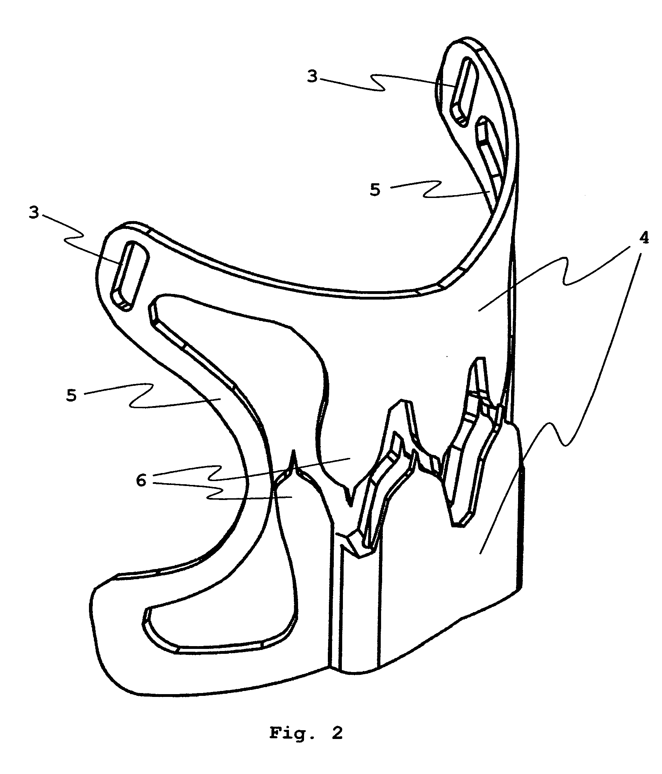

Im nachfolgenden werden einzelne Clipkonstruktionen anhand der Fig. 2 bis 4 näher erläutert.

Der Clip 1 gemäß der Fig. 2 zählt hinsichtlich seiner grundsätzlichen Konstruktion zum Stand der Technik und ist beispielsweise aus der WO 01/35832 der Anmelderin selbst bekannt.

Demzufolge besteht der Clip 1 aus einer maulartigen Klemmeinrichtung mit zwei gezahnten Kiefern 4, welche über zwei seitliche Scharniere bzw. durch flexible Ausformungen 5 auf- und zugeklappt werden können. Die Scharniere bzw. die flexiblen Ausformungen 5 sind dabei vorzugsweise aus federelastischen Bändern ausgebildet, welche beim Aufklappen der Kiefer 4 eine Federenergie speichern, die beim Freigeben der Kiefer 4 d.h. bei einem Auslösen der Scharniere bzw. der flexiblen Ausformungen 5 zu einem Zuschnappen der Kiefer 4 mit vorbestimmter Klemmkraft führt.

Im Einzelnen ist jeder Clip 1 einstückig aus einem Federblech gestanzt, indem aus dem Federblech ein Ring mit partiell unterschiedlicher Ringbreite herausgearbeitet wird. Zwei diametrisch gegenüberliegende Ringabschnitte mit großer Ringbreite bilden die beiden Kiefer 4, wohingegen die beiden dazwischenliegenden Ringabschnitte mit schmaler Ringbreite die Scharniere bzw. die flexiblen Ausformungen 5 ergeben. Die Kiefer 4 sind dadurch ausgebildet, indem die Ringabschnitte mit großer Ringbreite bogenförmig gewölbt werden, wobei die beiden Ringabschnitte mit schmaler Ringbreite um ihre Längsachse um ca. 90° verdreht (tordiert) werden um die Scharniere auszuformen. Durch diese bleibende Verformung des ausgestanzten Federblechs entsteht die Form einer Art Haifischmaul mit zwei aufeinander sich zu bewegenden Zahnreihen 6, welche durch Ausstanzen der Ringabschnitte mit großer Ringbreite gebildet werden.

An dieser Stelle sei darauf hingewiesen, dass die Konstruktion des Clips 1 gemäß Fig. 1 von dem vorstehend Beschriebenen abweichen kann. So ist es grundsätzlich möglich, die beiden Kiefer 4 getrennt voneinander auszubilden und diese über zwei seitliche Scharniere mit Scharnierstiften miteinander zu verbinden. Die Vorspannkraft, welche für den Klemmeingriff bzw. das Zuschnappen beider Kiefer 4 erforderlich ist, kann in diesem Fall durch eine externe Federeinrichtung (nicht dargestellt) erzeugt werden, welche im Bereich beider Scharniere 5 angeordnet ist. Auch ist es denkbar, anstelle einer eine Vorspannkraft erzeugenden Einrichtung, wie sie in Fig. 1 dargestellt ist, einen zusätzlichen Verschlussmechanismus anzuordnen, der beide Kiefer 4 in geschlossener Position arretiert. In diesem Fall müsste die Schließkraft extern mittels eines entsprechenden Werkzeugs auf die beiden Kiefer 4 aufgebracht werden, worauf dann der Verschlussmechanismus betätigt wird, um das Werkzeug anschließend wieder entfernen zu können.

Ein wesentliches konstruktives Merkmal des vorstehend beschriebenen Clips 1 oder Ankers gemäß Fig. 1 besteht in der eingangs kurz erwähnten Einhängvorrichtung zum Verbinden des Clips/Ankers 1 mit dem Band 2, Draht oder Schlauch.

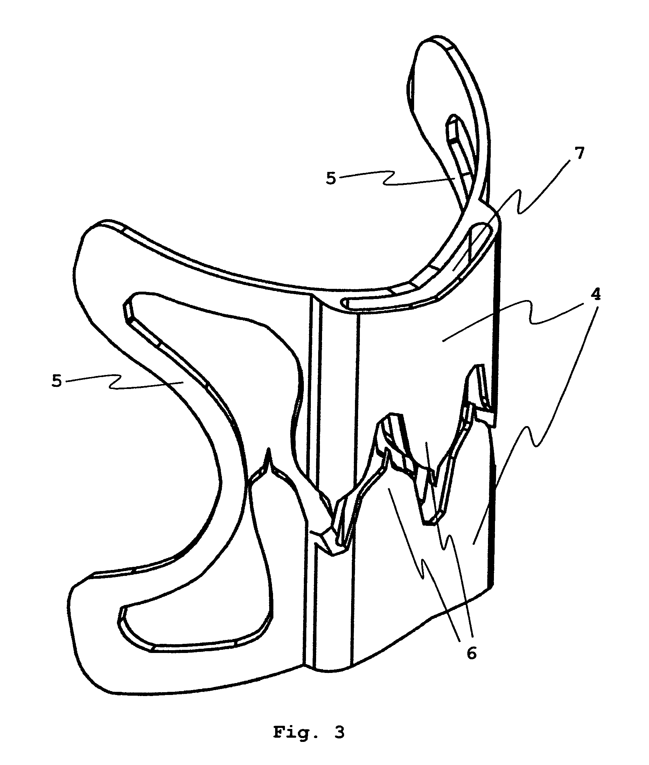

In der einfachsten Ausführungsform ist diese Einhängvorrichtung die Durchgangsbohrung 3 (siehe Fig. 2) durch einen der Kiefer 4 oder eine Öse 7 (siehe Fig. 3), welche beim Ausstanzen des Clips 1 oder bei Herstellung des Ankers zusätzlich ausgebildet wird und durch welche das Band 2, Draht oder Schlauch hindurchgezogen ist. Letztendlich kann auch die zwischen Scharnier 5 und Kiefer 4 sich ausbildende Ausnehmung selbst als Öse verwendet werden. Alternativ hierzu ist es aber auch möglich, den Clip 1 oder Anker mit einer lös-/öffenbaren Einhängvorrichtung (nicht gezeigt zusätzlich auszustatten, die ein nachträgliches Einhängen des Clips 1 oder Ankers an das bereits zu einem geschlossenen Ring geformte Band 2, Draht oder Schlauch ermöglicht. In diesem Fall kann die Einhängvorrichtung beispielsweise ein Schnapphaken oder dergleichen sein, welcher am Clip 1 oder Anker beispielsweise in der vorstehend beschriebenen Durchgangsbohrung 3 oder Öse befestigt ist. Auch ist es denkbar, Teile des Clips 1 oder Ankers, insbesondere die Scharniere 5 bzw. flexiblen Ausformungen des Clips 1 konstruktiv so zu gestalten, dass sich eine Einhängvorrichtung ergibt. Dies könnte beispielsweise dadurch erreicht werden, indem das Scharnier 5, bzw. die flexible Ausformung schnecken- bzw. schleifenförmig ausgebildet wird, wodurch sich selbsttätig eine im wesentlichen geschlossene Durchgangsöffnung zur Aufnahme des Bands oder einer weiteren lösbaren Einhängvorrichtung ergibt.

Die Funktionsweise der vorstehenden medizinischen Vorrichtung lässt sich wie folgt beschreiben:

Wie eingangs bereits ausgeführt wurde, stellt eine endoskopische Implantation einer medizinischen Vorrichtung insgesamt das für den Patienten verträglichste Verfahren dar. In diesem Fall muss die medizinische Vorrichtung von der Innenseite des Hohlorgans an diesem fixiert werden. Wie aus der Fig. 1 zu entnehmen ist, werden daher eine Anzahl der vorstehend beschriebenen Gewebeklammern 1, Clips oder Anker mittels eines nicht weiter dargestellten Endoskops in das Hohlorgan eingeführt und an vorbestimmten Stellen an der Organinnenseite platziert. Hiefür wird der jeweilige Clip 1 oder Anker an das Organgewebe herangeführt und die Vorspannfeder für ein Zuschnappen des Clips 1 oder Aufspannen des Ankers ausgelöst. Dieser klemmt oder hält daraufhin eine Gewebefalte zwischen seinen Kiefern 4 oder seinen Haken oder Nadeln mit einer vorbestimmten Klemm- oder Spreizkraft ein, wobei sich die Zähne 6, Haken, Nadeln oder Zacken jedes Kiefers 4 in das Gewebe bohren und dieses vorzugsweise durchdringen. Auf diese Weise wird jeder Clip 1 oder Anker in bestimmten Abständen zueinander an der Organinnenseite verankert und bildet somit für eine Zugkraft einen Einleitpunkt in das Organgewebe.

Anschließend wird das Band 2, Draht oder Schlauch mit der Einhängvorrichtung jedes Clips oder Ankers in Eingriff gebracht, d.h., das Band, Draht oder Schlauch wird durch die Durchgangsöffnung/Öse 7 an jedem Clip oder Anker hindurchgeführt oder der Clip wird über die öffenbare zusätzliche Einhängvorrichtung am Band, Draht oder Schlauch eingehängt.

Schließlich wird der vom Band, Draht oder Schlauch ausgebildete Ring- bzw. Schlaufendurchmesser verringert, indem im Fall des Bands oder Drahts dieses an seinen freien Enden zusammengezogen wird. Im Fall eines Schlauchs wird dieser evakuiert oder gefüllt, wodurch sich der Durchmesser des zu einem Ring oder Schlaufe vorgeformten Schlauchs verändert.

Durch das Verringern des Ring- bzw. Schlaufendurchmessers wird auf die im Organgewebe verankerten Clips oder Anker eine Zugkraft aufgebracht, welche, dem Band, Draht oder Schlauch nacheilend ein Zusammenziehen des Organs am betreffenden Querschnittsbereich bewirkt.

Durch die vorstehend beschriebene Vorrichtung sowie die hiermit verbundenen Funktionen ergeben folgende Vorteile gegenüber dem Stand der Technik:
- Wie bereits ausgeführt wurde, kann die Vorrichtung endoskopisch, gegebenenfalls mit Unterstützung durch eine Instrumentierung von der Außenseite des Organs her über ein chirurgischen Zugang in das Organ eingesetzt werden, und ist somit für den Patienten wenig belastend.
- Da die Kraftanlenkpunkte nicht durch Vernähen sondern per Gewebeklemme/Clip/Anker erfolgt, ist die Installation der Vorrichtung in kurzer Zeit zu bewerkstelligen, was sich sowohl für den Patienten, als auch die Behandlungskosten günstig auswirkt.
- Die Vorrichtung, kann so ausgebildet werden, daß sie reversibel einsetzbar ist, d.h. sie kann dann auf einfache Weise wieder entfernt und somit das betreffende Organ in seine ursprüngliche Form und Größe rückversetzt werden.
- Wird die Vorrichtung zur Verringerung des Magenvolumens eingesetzt, hat das innenliegende Band, Draht oder Schlauch den zusätzlichen Effekt eines Art Strömungswiderstands. D.h. das innenliegende Band, Draht oder Schlauch hält aufgenommene Nahrung zurück, wodurch sich diese oberhalb der Vorrichtung und der hierdurch bewirkten Einschnürung des Magens aufstaut. Dadurch wird schnell ein Völlegefühl beim Patienten ausgelöst und so die Gesamtmenge an aufgenommener Nahrung pro Portion reduziert.
- Wird die Vorrichtung zur Reduzierung von Reflux aus dem Magen in die Speiseröhre eingesetzt, hat das entsprechende Band die Funktion durch Stützen der Magenwand im Bereich des Mageneingangs den Rückfluß von Mageninhalt in die Speiseröhre zu verringern.

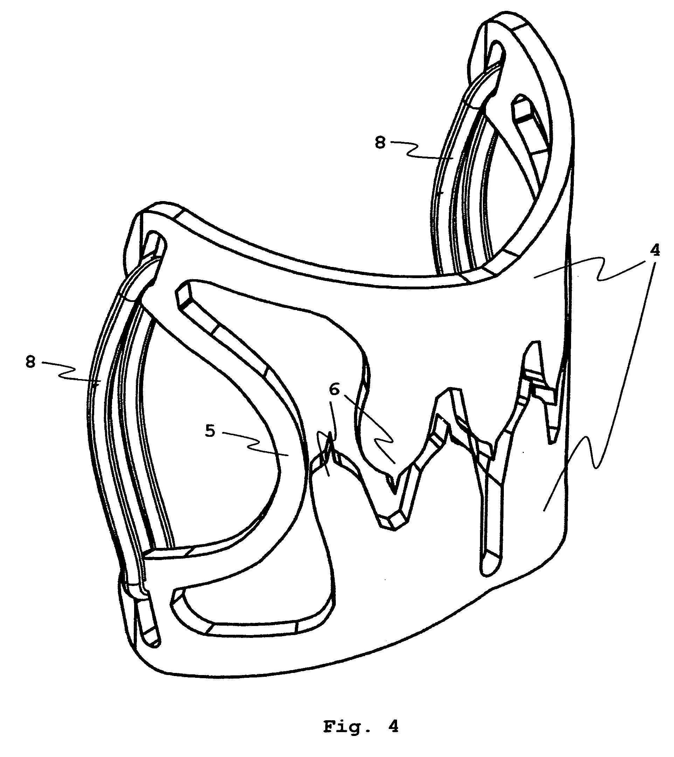

In den Fig. 2 bis 4 sind unterschiedliche Varianten für einen erfindungsgemäßen Clip 1 dargestellt mit unterschiedlichen Zahnreihen und Federvorspannungen. Insbesondere Gemäß der Fig. 4 ist der dort gezeigte Clip 1 mit einer zusätzlichen Vorspanneinrichtung 8 in Form eines Gummizugs ausgestattet, der die beiden Kiefer 4 in Öffnungsrichtung vorspannt. Die übrige Konstruktion des Clips 1 gemäß der Fig. 4 entspricht der des Clips gemäß Fig. 2.

Die zusätzliche Vorspanneinrichtung 8 hat die Funktion, den Clip 1 selbsttätig in Offenposition zu halten, wobei die Schließkraft der Scharniere 5 erst dann die Kiefer in Schließposition wirksam presst, wenn die Zuklappbewegung der Kiefer einen Schwellpunkt überschritten hat.

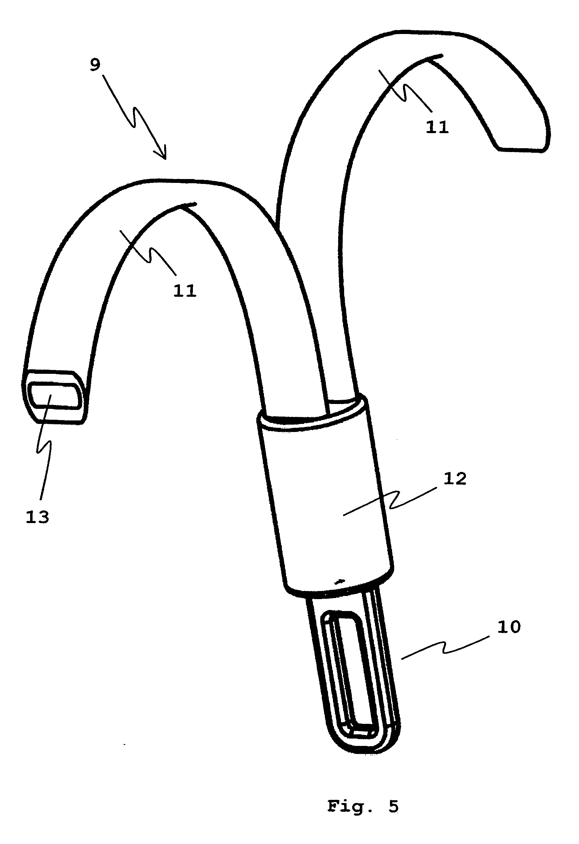

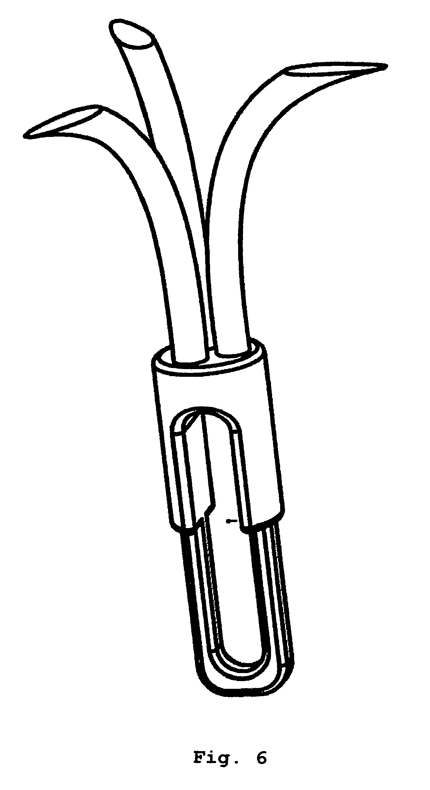

In den Fig. 5 und 6 sind nicht erfindungsgemäße Zuganker, welche grundsätzlich zum Stand der Technik gehören und die beispielsweise aus der DE 10159470 der Anmelderin selbst bekannt sind, als alternative Konstruktionen zu dem erfindungsgemäßen Clip 1 dargestellt, im wesentlichen bestehend aus einer Anzahl von Haken 9 und einer vorzugsweise ösenförmigen Einhängvorrichtung 10 für das Band, Draht oder Schlauch.

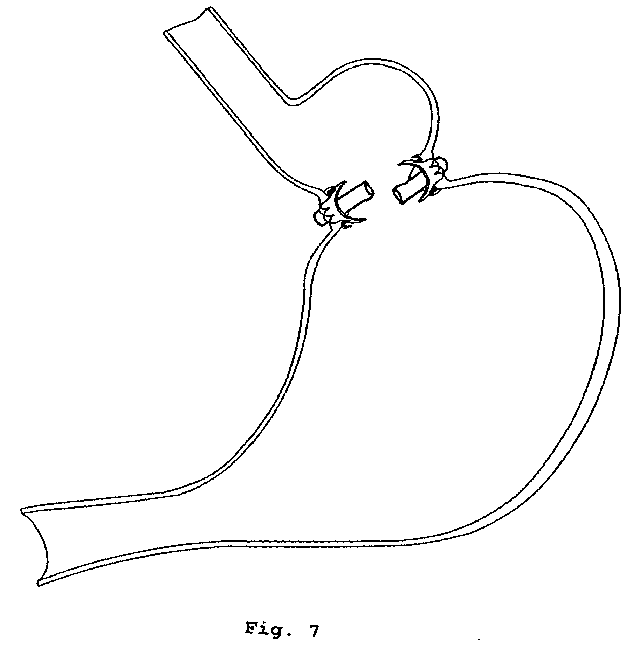

Gemäß der Fig. 5 besteht der Haken 9 aus zwei bogenförmig gekrümmten Armen 11, welche an einem Ende an einem Basisstück 12 zusammengefasst sind. Vorzugsweise sind die gekrümten Arme 11 röhrenförmig ausgebildet, wobei sich der innere Längskanal 13 an den freien Enden der Arme 11 nach Außen öffnet. Gemäß der Fig. 7 weist der Haken 9 im Unterschied zum haken gemäß der Fig. 5 drei bogenförmig gekrümmte Arme 11 auf, welche in diesem Fall jedoch aus einem Vollmaterial geformt sind.

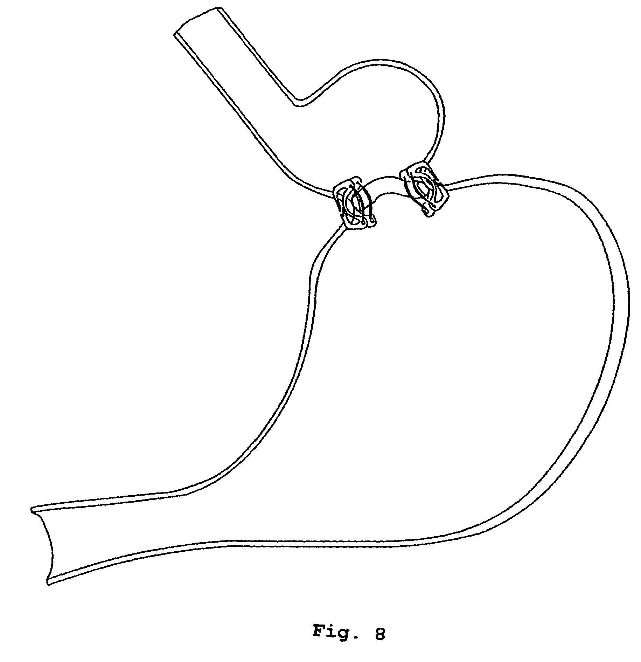

In den Fig. 7 und 8 ist die medizinische Vorrichtung gemäß dem ersten Ausführungsbeispiel unter Verwendung der Clips gemäß der Fig. 2 und 4 dargestellt. Bei dieser besonderen Anwendung der medizinischen Vorrichtung sind jeweils zwei diametral angeordnete Clips vorgesehen, welche jeweils von der Organinnenseite her an der Organwand fixiert sind. Das Band ist an den jeweiligen Ösen/Durchgängen der Clips eingehängt und durchquert dabei den Organhohlraum auf direktem Weg. Letzteres ist insbesondere in Fig. 8 dargestellt.

Der Vorteil dieser Ausrichtung des Bands besteht darin, dass hierdurch eine Art Strömungswiderstand gebildet wird, der verhindert, dass aufgenommene Nahrung das Hohlorgan zu schnell durchläuft. D.h. das Band ist so ausgerichtet, dass es ein Aufstauen der Nahrung bewirkt, wodurch schneller ein Sättigungsgefühl beim Patienten ausgelöst wird.

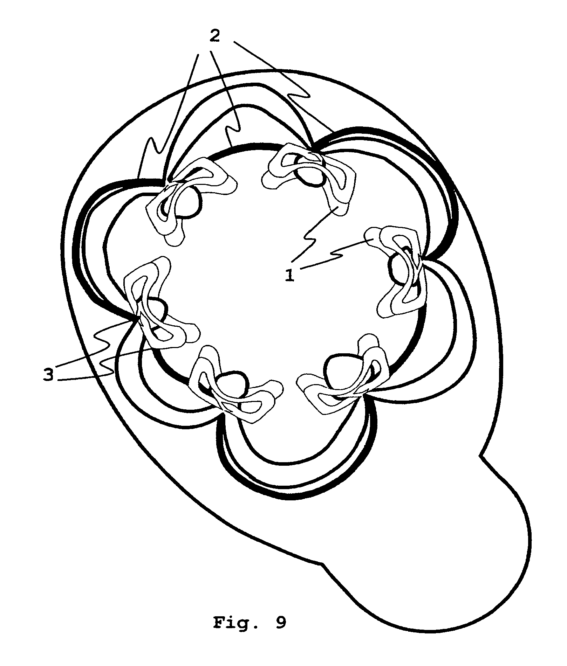

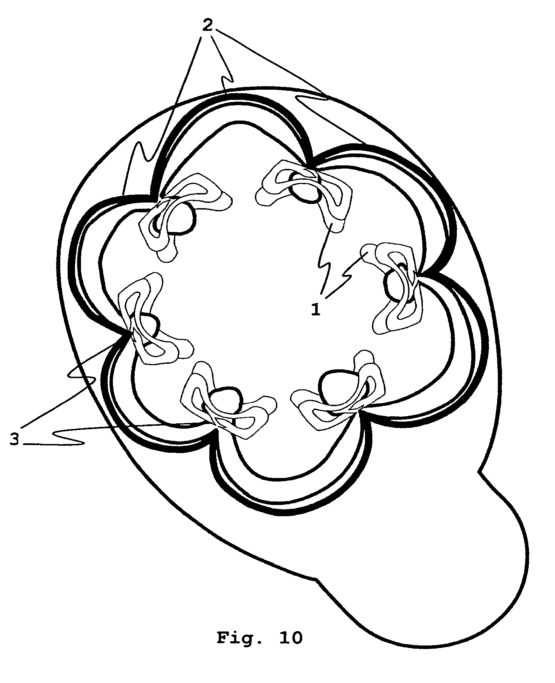

In den Fig. 9 und 10 ist ein zweites bevorzugtes Ausführungsbeispiel der Erfindung dargestellt.

Auch bei dem zweiten Ausführungsbeispiel besteht die medizinische Vorrichtung gemäß der Erfindung aus einer Anzahl von Gewebeklemmen 1 bzw. Clips oder Anker und einem Band, Draht oder Schlauch 2, auf das die Clips 1 oder Anker aufgereiht sind. In diesem Fall sind die Clips 1 oder Anker jedoch nicht als Zuganker, sondern lediglich als Positionierhalterungen ausgebildet. Prinzipiell entspricht der konstruktive Aufbau der Klemmen 1 gemäß dem zweiten Ausführungsbeispiel jenem gemäß dem vorstehend beschriebenen Ausführungsbeispiel. Indessen ist es beim zweiten Ausführungsbeispiel vorgesehen, das Band 2, Draht oder Schlauch als offenen Ring oder Schlaufe auszubilden, wobei an einem Ende des Bands 2, Draht oder Schlauchs eine Nadel (nicht weiter gezeigt) angeordnet ist. Diese Nadel dient dabei nicht nur zur Erleichterung des Einfädelns des Bands 2, Drahts oder Schlauchs in die Durchgangsöffnungen/Ösen 3 der Klemmen 1, sondern auch zum Durchstoßen der Organwand, wie dies in der Fig. 9 und 10 dargestellt ist.

In diesem Fall wird das Band 2, Draht oder Schlauch von der Organinnenseite her endoskopisch im unmittelbaren Bereich jedes Clips 1 nach dessen Einfädeln in der entsprechenden Öse 3 durch die Organwand geführt und bis zum jeweils benachbarten Clip 1 oder Anker um das Organ an dessen Außenseite herumgelegt. Anschließend wird das Band 2 Draht oder Schlauch wieder durch die Organwand hindurch nach innen zurückgeholt und erneut an dem entsprechenden Clip 1 eingefädelt. Beim Zusammenziehen der beiden freien Enden des Bands 2, Drahts oder Schlauchs und anschließenden Fixieren (durch Verknoten, Verschweißen oder mittels einer Fixiereinrichtung wie eine Klemmhülse) wird auf die Organaußenseite ähnlich einer Manschette ein radial gleichmäßiger Druck ausgeübt, der ein Einschnüren des Organs bewirkt. Dabei kann das Band abwechselnd an der Außen- und Innenseits des Organs geführt sein, wie dies in der Fig. 9 gezeigt ist, oder im wesentlichen vollständig das Organ außenseitig umgeben, wie dies in der Fig. 10 angedeutet ist.

In beiden Fällen haben die Klemmen 1 lediglich die Funktion, ein Abrutschen des Bands 2, Draht oder Schlauchs zu verhindern und somit die Einschnürstelle exakt zu halten. In sofern kann die Vorspannkraft auf die beiden Kiefer 4 des Clip 1 bzw. auf die Haken des Ankers gegenüber dem ersten Ausführungsbeispiel deutlich geringer sein, sodass auch die Federeinrichtung selbst kleiner ausgestalten werden kann. Der Vorteil dieser zweiten Ausgestaltung der medizinischen Vorrichtung gegenüber dem ersten Ausführungsbeispiel liegt in den folgenden Effekten:
- Durch die geringere erforderliche Klemm- bzw. Spannkraft des Clip bzw. die geringere Spreizkraft des Ankers wird das Organgewebe an den jeweiligen Verankerungspunkten weniger stark mechanisch belastet.
- Die Einschnürkraft wird nicht punktuell wie beim ersten Ausführungsbeispiel sondern linear auf das Organ aufgebracht. Hierdurch ergibt sich eine homogenere Kraftverteilung.

Gemäß einer Variante des vorstehend beschriebenen zweiten Ausführungsbeispiels besteht die abgewandelte medizinische Vorrichtung gemäß der Erfindung aus einer Anzahl von Gewebeklemmen bzw. Clips oder Anker und einem Band, Draht oder Schlauch, auf das die Clips oder Anker aufgereiht sind. In diesem Fall sind die Clips oder Anker wie im zweiten Ausführungsbeispiel als Positionierhalterungen ausgebildet. Prinzipiell entspricht der konstruktive Aufbau der Klemmen gemäß der Abwandlung jenem gemäß dem vorstehend beschriebenen zweiten Ausführungsbeispiel. Indessen ist es bei der Abwandlung des zweiten Ausführungsbeispiels vorgesehen, das Band, Draht oder Schlauch als offenen Ring oder Schlaufe auszubilden.

In diesem Fall werden die Clips bzw. Anker endoskopisch an die entsprechenden Positionen von der Organinnenseite her platziert und das Band, Draht oder Schlauch von der Organaußenseite her laparoskopisch, minimal invasiv oder offen chirurgisch durch die ringförmigen Ösen der Clips bzw. des Ankers durchgefädelt, welche komplett die Organwand durchstoßen haben. D.h. in diesem Fall befinden sich die Ösen bzw. Durchgangsbohrungen an einer Stelle des Clip welche nach dessen Befestigung an der Organwand an der Organaußenseite zuliegen kommt. Dies ist insbesondere in der Fig. 10 dargestellt.

Beim Zusammenziehen der beiden freien Enden des Bandes, Drahts oder Schlauchs und anschließendem Fixieren (durch Verknoten oder mittels einer Fixiereinrichtung wie eine Klemmhülse) wird auf die Organaußenseite ähnlich einer Manschette ein radial gleichmäßiger Druck ausgeübt, der ein Einschnüren des Organs bewirkt.

Auch in diesem Fall haben die Klemmen bzw. Anker lediglich die Funktion ein Abrutschen des Bands, Draht oder Schlauchs zu verhindern und somit die Einschnürstelle exakt zu halten. In sofern weisen die beiden Kiefer des Clips bzw. des Ankers gegenüber dem ersten und zweiten Ausführungsbeispiel eine ringförmige Öse am Kiefer (insbesondere an den Zähnen) oder der Haken auf, so dass das Band, Draht oder Schlauch durchgefädelt werden kann. Der Vorteil dieser dritten Ausgestaltung der medizinischen Vorrichtung gegenüber dem ersten Ausführungsbeispiel liegt in den folgenden Effekten:
- Das Band, Draht oder Schlauch wird vollkommen an der Außenseite des Organs platziert und kann die Einschnürkraft noch besser als im ersten und zweiten Ausführungsbeispiel radial auf das Organ aufgebringen. Hierdurch wird die Einsschnürkraft homogener auf die äußere Organwand aufgebracht.
- Durch diese Vorrichtung ist zudem das Einfädeln durch einen minimal invasiven bzw. offenen chirurgischen Eingriff einer weiteren Möglichkeit für den Arzt gegeben. Diese Vorrichtung kann somit das Gewebe zwischen den Clips oder Anker besser komprimieren bzw. radial festklemmen.

Abschließend sei darauf hingewiesen, dass die Ausgestaltung des Clips oder Ankers in Abhängigkeit von dessen Einsatzzweck und -ort unterschiedlich sein kann. So können unterschiedliche Zähne-, Haken oder Nadelformen die Erzeugung eines Unter- oder Überbisses der beiden Kiefer sowie unterschiedliche Verankerungsarten, wie einfaches Einklemmen von Gewebe bis hin zu vollständiges Durchdringen der Organwand und Verkrallen der Zähne, Haken oder Nadeln vorgesehen sein. Auch sind Material und Beschichtung der Clips oder Anker und des Bandes, Drahts oder Schlauchs beliebig wählbar. So kann jeder Clip, Anker, Band, Draht oder Schlauch mit einem besonderen Überzug versehen sein, der ein Verwachsen mit dem Organgewebe anregt, um so eine größere Zugkraft in das Organgewebe einleiten zu können. Oder aber der Clip oder der Anker, das Band, der Draht oder der Schlauch sind aus einem sich selbst auflösenden Material, wodurch ein Entfernen der Vorrichtung nach einer vorbestimmten Zeitperiode entfallen kann.

Die vorliegende Erfindung betrifft im Wesentlichen eine medizinische Vorrichtung zur Veränderung der Form menschlicher Hohlorgane. Diese Vorrichtung hat unter anderem eine Gewebeklammer oder Clip oder alternativ einen Anker, der mit dem Organgewebe in Klemmeingriff bzw. in Verhakungseingriff bringbar ist, um einen Halte-/Krafteinleitpunkt am Organ zu bilden. Der Clip hat/bildet eine Aufnahme oder Halterung für ein flexibles Band, Draht oder Schlauch, mittels welchem eine die Formänderung bewirkende Kraft auf das Organ vorzugsweise über den Clip aufbringbar ist.

Neben den beschriebenen Ausführungen zur Verwendung der erfindungsgemäßen Vorrichtung am Magen, sind auch Ausführungen für die Verwendung in anderen Abschnitten des Verdauungstraktes möglich, so z.B. im Darm.

Auch an anderen Hohlorganen, als dem Verdauungstraks kann die erfindungsgemäße Vorrichtung zur Veränderung des Durchmessers oder der Form eingesetzt werden. Dies betrifft z.B. das Blutgefäßsystem oder die Harnwege.

Die vorliegende Erfindung betrifft eine medizinische Vorrichtung zur Veränderung der Form menschlicher Hohlorgane gemäß den Merkmalen des Anspruchs 1.

## Patentansprüche

1. Medizinische Vorrichtung zur Veränderung der Form Menschlicher Hohlorgane mit zumindest einer Gewebeklammer oder Clip (1) bestehend aus zumindest zwei Kiefern (4), die über Scharniere oder eine flexible Ausformung (5) relativ zueinander verschwenkbar miteinander verbunden sind, sodass der Clip mit dem Organgewebe in einen Klemmeingriff zwischen seinen Kiefern bringbar ist, um einen Halte-/Krafteinleitungspunkt am Organ zu bilden, **gekennzeichnet durch** ein flexibles Band (2), Draht oder Schlauch, der **durch** eine in Form einer Durchgangsbohrung (3) an zumindest einem der Kiefer ausgebildete Aufnahme oder Einhängevorrichtung geführt ist und mittels dem eine die Formänderung bewirkende Kraft auf das Organ zumindest teilweise über den Clip aufbringbar ist.

2. Medizinische Vorrichtung nach einem der vorstehenden Ansprüche, **dadurch gekennzeichnet, dass** der Kiefer (4) Zähne (6), Haken oder Nadeln aufweist und dass die Zähne, Haken oder Nadeln des Kiefers zwischen den Scharnieren oder flexiblen Ausformungen angeordnet sind.

3. Medizinische Vorrichtung nach einem der vorstehenden Ansprüche, **dadurch gekennzeichnet, dass** die Zähne (6) Haken oder Nadeln der Kiefer entsprechende Profile vorweisen, die zueinander verhakbar sind.

4. Medizinische Vorrichtung nach einem der vorstehenden Ansprüche, **dadurch gekennzeichnet, dass** das flexible Band, Draht oder Schlauch aus einem schwer dehnbaren oder einem elastischen Material besteht.

5. Medizinische Vorrichtung nach einem der vorstehenden Ansprüche, **dadurch gekennzeichnet, dass** das Band, Draht oder Schlauch aus einem Metall, einer Formgedächtnislegierung oder aus Kunststoff besteht.

6. Medizinische Vorrichtung nach einem der vorstehenden Ansprüche, **dadurch gekennzeichnet, dass** der Schlauch durch eine Anschlusseinrichtung mit einem körperverträglichen Fluid befüllbar oder evakuierbar ist.

7. Medizinische Vorrichtung nach einem der vorstehenden Ansprüche, **dadurch gekennzeichnet, dass** das Band, Draht oder Schlauch zu einem offenen oder geschlossenen Ring oder Schlaufe geformt ist, wobei im Fall eines offenen Rings bzw. Schlaufe die beiden freien Enden des Bands, Drahts oder Schlauchs mittels eines Verschlusses unter Verstellung des aktuellen Ring- bzw. Schlaufendurchmesser miteinander verbindbar sind.

8. Medizinische Vorrichtung nach einem der vorstehenden Ansprüche, **dadurch gekennzeichnet, dass** das Band, Draht oder Schlauch an einem freien Ende mit einer Nadel versehen ist.

9. Medizinische Vorrichtung nach einem der vorstehenden Ansprüche, **dadurch gekennzeichnet, dass** der Clip und das Band, Draht oder Schlauch mit einem die Verwachsung mit einem organischen Gewebe fördernden oder hemmenden Überzug versehen ist.

10. Medizinische Vorrichtung nach einem der vorstehenden Ansprüche, **dadurch gekennzeichnet, dass** der Clip, das Band, Draht oder Schlauch aus einem solchen Material besteht, welches sich nach einer vorgegebenen Zeit in Flüssigkeit auflöst.

## Claims

1. A medical device for changing the shape of hollow organs in the human body with at least one clip comprising at least two jaws that are pivotably connected to each other by joints or a flexible recess so that the clip is fixable to organ tissue by means of clamping between its at least two jaws in order to provide a fixation point or point of transmission of force to the organ, **characterized by**
one of a flexible band, wire or tube guided through a receiving element portion or hooking-in device configured in form of a through-hole on at least one jaw and by means of which a force causing a change of shape can be applied to the organ at least partially via the clip.

2. A medical device according to claim 1, **characterized in that** the jaw comprises teeth, hooks or needles and that the teeth, hooks or needles of the jaw are arranged between the joints or flexible recesses.

3. A medical device according to claim 1 or 2, **characterized in that** the teeth, hooks or needles of the jaws comprise corresponding profiles that can be interlocked with each other.

4. A medical device according to any one of claims 1 to 3, **characterized in that** the flexible band, wire or tube is made form a hardly expandable or an elastic material.

5. A medical device according to any one of claims 1 to 4, **characterized in that** the band, wire or tube is made from a metal, a shape memory alloy or a plastic.

6. A medical device according to any one of claims 1 to 5, **characterized in that** the tube can be inflated or deflated with a biocompatible fluid by means of a connecting device.

7. A medical device according to any one of claims 1 to 6, **characterized in that** the band, wire or tube is formed to an open or closed ring or loop, wherein in case of an open ring or loop the two loose ends of the band, wire or tube can be connected to each other by means of a closing mechanism which enables the adjustment of the current ring or loop diameter.

8. A medical device according to any one of claims 1 to 7, **characterized in that** the band, wire or tube is provided with a needle at one loose end.

9. A medical device according to any one of claims 1 to 8, **characterized in that** the clip as well as the band, wire or tube are provided with a coating that enhances are inhibits intergrowth with an organ tissue.

10. A medical device according to any one of claims 1 to 9, **characterized in that** the clip, band, wire or tube are made from such material that dissolves in the body after elapse of a predetermined period of time.

## Revendications

1. Dispositif médical pour la modification de la forme d'organes creux humains, comportant au moins une agrafe tissulaire ou un clip (1) constitué d'au moins deux mâchoires (4) qui sont reliées l'une à l'autre par des charnières ou un façonnage flexible (5) pouvant basculer l'une par rapport à l'autre de sorte que le clip avec les tissus organiques puisse être amené à une opération de serrage entre ses mâchoires pour former sur l'organe, un point de maintien / d'introduction de force, **caractérisé par** une bande (2), un fil ou un ruban flexible qui est introduit dans un évidement ou un dispositif de suspension formé sous la forme d'un alésage traversant (3) sur au moins l'une des mâchoires et peut être appliqué au moins partiellement sur le clip au moyen de la force entraînant la modification de forme.

2. Dispositif médical selon l'une des revendications précédentes, **caractérisé en ce que** la mâchoire (4) présente des dents (6), des crochets ou des aiguilles et que les dents, les crochets ou les aiguilles de la mâchoire sont disposés entre les charnières ou les évidements flexibles.

3. Dispositif médical selon l'une des revendications précédentes, **caractérisé en ce que** les dents (6), crochets ou aiguilles de la mâchoire présentent des profils correspondants qui peuvent s'engrener les uns dans les autres.

4. Dispositif médical selon l'une des revendications précédentes, **caractérisé en ce que** la bande, le fil ou le ruban flexible sont constitués d'un matériau difficilement extensible ou d'un matériau élastique.

5. Dispositif médical selon l'une des revendications précédentes, **caractérisé en ce que** la bande, le fil ou le ruban sont constitués de métal, d'alliage à mémoire de forme ou de matière plastique.

6. Dispositif médical selon l'une des revendications précédentes, **caractérisé en ce que** le ruban peut être rempli d'un fluide acceptable sur le plan corporel ou évacué par un dispositif de raccordement.

7. Dispositif médical selon l'une des revendications précédentes, **caractérisé en ce que** la bande, le fil ou le ruban est formé(e) en un anneau ou une boucle ouvert(e) ou fermé(e), dans le cas d'une boucle ou d'un anneau ouvert(e), les deux extrémités libres de la bande, du fil ou de la boucle pouvant être reliées l'une à l'autre au moyen d'un joint en faisant varier le diamètre actuel de l'anneau ou de la boucle.

8. Dispositif médical selon l'une des revendications précédentes, **caractérisé en ce que** la bande, le fil ou le ruban sont dotés d'une aiguille à une extrémité libre.

9. Dispositif médical selon l'une des revendications précédentes, **caractérisées en ce que** le clip et la bande, le fil ou le ruban sont dotés d'un revêtement favorisant ou inhibant l'association avec un tissu organique.

10. Dispositif médical selon l'une des revendications précédentes, **caractérisé en ce que** le clip, la bande, le fil ou le ruban sont constitués d'un qui se dissout dans un fluide après un certain temps.
